# EUROPÄISCHE PATENTSCHRIFT

(11) **EP 3 295 170 B1**
(45) Veröffentlichungstag und Bekanntmachung des Hinweises auf die Patenterteilung: **29.03.2023**
(21) Anmeldenummer: 16725360.8
(22) Anmeldetag: 11.05.2016
(51) Int. Cl.: G01N 33/497, G01N 33/98, C07C 51/16

(54) **FESTBETTREAKTOR, VERFAHREN ZUR HERSTELLUNG EINES FESTBETTREAKTORS UND VERWENDUNG EINES FESTBETTREAKTORS**
FIXED-BED REACTOR, METHOD FOR PRODUCING A FIXED-BED REACTOR, AND USE OF A FIXED-BED REACTOR
RÉACTEUR À LIT FIXE, PROCÉDÉ DE FABRICATION D'UN RÉACTEUR À LIT FIXE ET UTILISATION D'UN RÉACTEUR À LIT FIXE

(30) Priorität: 12.05.2015 DE 102015005943
(43) Veröffentlichungstag der Anmeldung: 21.03.2018
(73) Patentinhaber: Dräger Safety AG & Co. KGaA, 23560 Lübeck (DE)
(72) Erfinder: BOECKMANN, Jan, 23569 Lübeck (DE); BRETT, Stefanie, 23558 Lübeck (DE)
(74) Vertreter: Guthöhrlein, Gerhard
(86) Internationale Anmeldenummer: PCT/EP2016/000769
(87) Internationale Veröffentlichungsnummer: WO 2016/180532

(56) Entgegenhaltungen:
- EP-A1- 2 698 201
- EP-A1- 2 698 201
- US-A- 4 791 065
- US-A- 5 834 571
- US-A- 5 834 571
- Lynch Megan: "PURIFIED PHOSPHORIC ACID H 3 PO 4 TECHNICAL INFORMATION BULLETIN", , 14 April 2021 (2021-04-14), pages 1-33, XP093001931, Retrieved from the Internet: URL:https://isolab.ess.washington.edu/reso urces/H3PO4.pdf [retrieved on 2022-11-24]

## Beschreibung

Die vorliegende Erfindung betrifft ein Festbett entsprechend Anspruch 1, ein Verfahren zur Herstellung eines solchen Festbettes sowie die Verwendung eines solchen Festbettes als Festbettreaktor, insbesondere die Verwendung eines solchen Festbettes bzw. Festbettreaktors zum Nachweis von flüchtigen organischen Verbindungen, z.B. alkoholischen Verbindungen.

Festbettreaktoren sind grundsätzlich bekannt. Dabei handelt es sich typischerweise um eine feste Schüttung von Partikeln, durch welche ein Fluid hindurchströmen kann, (Festbett), die in einem entsprechenden Gehäuse angeordnet ist. Eine feste Schüttung ist dabei eine Schüttung bei der die Partikel in optimal dichter Packung entsprechend ihrer Form vorliegen und so wenig wie möglich Bewegungsspielraum haben. Gleichzeitig können die Partikel in dieser festen Schüttung natürlich von einem Gasstrom durchströmt werden. Die Partikel dienen üblicherweise als Träger für wenigstens einen reaktiven Stoff. Der reaktive Stoff ist typischerweise so beschaffen, dass er mit einem bestimmten Stoff, der in dem Fluid vorhanden ist, reagieren kann, wenn das Fluid an dem jeweiligen Partikel vorbeiströmt. Solche Festbettreaktoren werden zum Beispiel als Gasmessröhrchen (Röhrchen) eingesetzt, beispielsweise in Form der unter der Marke "Dräger Röhrchen"^{®} vertriebenen Produkte der Anmelderin. Ein solches Gasmessröhrchen besteht beispielsweise aus einem durchsichtigen Gehäuse mit einem ersten und einem zweiten Ende, so dass ein Fluid durch das Gehäuse hindurch strömen kann. In dem Gehäuse befindet sich dann ein entsprechendes Festbett, das z.B. mit Hilfe eines Drahtgewebes oder dergleichen im Gehäuse fixiert sein kann.

Neben verschiedensten Anwendungen im industriellen Bereich - beispielsweise zum Nachweis von verschiedenen kurzkettigen alkoholischen Verbindungen, wie etwa Methanol, Ethanol, Propanol oder Butanol - ist ein bekanntes Anwendungsbeispiel für solche Gasmessröhrchen der Nachweis von Atemalkohol. Dabei wird das Gasmessröhrchen in eine Vorrichtung eingesetzt, in welche ein Proband hineinpusten kann. Der Atem des Probanden durchströmt dann das Röhrchen. Das Festbett ist in diesem Fall so ausgebildet, dass eine Reaktion mit Alkohol stattfinden kann. Ist eine entsprechende Menge Alkohol im Atem des Probanden vorhanden, so findet eine Farbumschlagsreaktion im Bereich des Festbettreaktors statt, die entsprechend optisch erkennbar ist.

Die Farbumschlagsreaktion beruht typischerweise auf einer Redoxreaktion unter Beteiligung von Chromsalzen, beispielsweise eine Redoxreaktion, während der Chrom(VI) zu Chrom(III) reduziert wird. Ein solcher Ethanol sensitiver Feststoff ist bereits in der US 4791065 offenbart.

Ein Nachteil solcher Reaktionen ist jedoch das relativ hohe Gefährdungspotential für Mensch und Umwelt durch viele der grundsätzlich für einen solchen Nachweis geeigneten Chrom-Verbindungen, insbesondere anorganische Chromverbindungen. Eine industrielle Verarbeitung von anorganischen Chromverbindungen ist daher häufig nur unter Beachtung sehr hoher Sicherheitsstandards und unter Ergreifung gesteigerter Schutzmaßnahmen möglich. Die Herstellung von Festbettreaktoren, die solche Verbindungen enthalten, kann sich mithin sehr aufwändig und kostenintensiv gestalten.

Ausgehend davon ist es Ziel der Erfindung, diese und weitere Nachteile des Standes der Technik zu überwinden und einen verbesserten Festbettreaktor zu schaffen. Dieser soll kostengünstig und einfach herzustellen sein.

Zur Lösung dieser Aufgabe schlägt die Erfindung einen Festbettreaktor entsprechend Anspruch 1, sowie ein Verfahren zur Herstellung eines Festbettes für einen solchen Festbettreaktor entsprechend Anspruch 6, ein Gasmessröhrchen entsprechend Anspruch 11 mit einem solchen Festbett und die Verwendung der metallorganischen Pyridiniumverbindungen in einem Festbettreaktor entsprechend den Ansprüchen 12 und 13 vor. Ausgestaltungen sind jeweils Gegenstand der abhängigen Ansprüche.

Bei einem Festbett für einen Festbettreaktor, wobei das Festbett einen partikelförmigen Träger und wenigstens einen reaktiven Stoff aufweist und wobei der Träger eine Silikaverbindung ist, sieht die Erfindung vor, dass der reaktive Stoff eine metallorganische Pyridinverbindung ausgewählt aus Pyridiniumchlorochromat, Pyridiniumdichromat und Bispyridinchromtrioxid ist.

Ein Festbettreaktor mit einem solchen Festbett kann mit großem Vorteil für verschiedene Zwecke eingesetzt werden. So ist vorstellbar, dass das Festbett als Nachweisreagenz für eine Substanz dient, die in einem Gasstrom vorhanden ist, der durch den Festbettreaktor hindurchgeleitet wird. Mit anderen Worten, das Festbett kann als eine Indikatorschicht dienen. Ist in dem Gasstrom die fragliche Substanz enthalten, so kann es durch die Reaktion dieser Substanz mit dem an das Festbett gebundenen reaktiven Stoff zu einer optisch nachweisbaren Reaktion kommen. Mit Hilfe des erfindungsgemäßen reaktiven Stoffes ist in diesem Zusammenhang vor allem der Nachweis von alkoholischen Substanzen besonders gut möglich.

Es ist auch denkbar, dass der Festbettreaktor zur Synthese von Substanzen verwendet wird, wobei das Festbett den reaktiven Stoff bereitstellt und die umzusetzenden Edukte mithilfe eines Gasstromes zum reaktiven Stoff geleitet werden. Der reaktive Stoff ist dabei erfindungsgemäß an den Träger gebunden, sodass die Umsetzung der Edukte an der Oberfläche der partikelförmigen Träger stattfindet. Die dabei entstehenden Produkte werden mit dem abströmenden Gasstrom aus dem Festbettreaktor wieder freigesetzt. Dies bietet den besonderen Vorteil, dass der reaktive Stoff anschließend nicht mehr vom Produkt abgetrennt werden muss. Mit Hilfe des erfindungsgemäßen reaktiven Stoffes ist in diesem Zusammenhang insbesondere die Synthese von Formaldehyd oder Acetaldehyd besonders gut möglich. Eine so hergestellte Substanz kann zum Beispiel als Reagenz in einer weiteren, nachfolgenden Indikatorschicht dienen. So ist es zum Beispiel denkbar, dass in einem Gehäuse eines Festbettreaktors eine 1. Schicht, in welcher Formaldehyd hergestellt wird, und eine 2. Schicht, in welcher das Formaldehyd als Reagenz für einen Nachweis dient, angeordnet sind.

Man erkennt insofern, dass es im Sinne der vorliegenden Erfindung besonders vorteilhaft ist, wenn der reaktive Stoff die metallorganische Pyridinverbindung ist. Solche Verbindungen können insbesondere bei einer Reaktion mit Alkoholen einen deutlichen Farbumschlag zeigen. Gleichzeitig kann es bei dieser Reaktion zur Freisetzung von Formaldehyd und/oder Acetaldehyd kommen. Mithilfe eines erfindungsgemäßen Festbettreaktors ist mithin sowohl der Nachweis von Alkoholen in einem Gasgemisch, etwa in einem Atemgasstrom, als auch die Synthese von Formaldehyd und/oder Acetaldehyd möglich. Gleichzeitig bieten die metallorganischen Pyridinverbindungen den Vorteil, dass sie vielfach wesentlich weniger umweltgefährdend oder gar giftig als es beispielsweise anorganische Chromverbindungen sind. Zudem konnte überraschenderweise beobachtet werden, dass sie in ihrer Redox-Aktivität auch noch deutlich effektiver sein können als anorganische Chromverbindungen.

In einer bevorzugten Ausführungsform kann ein erfindungsgemäßer Festbettreaktor mithin ein Röhrchen zur Bestimmung von Atemalkohol sein. Denkbar ist auch, dass ein erfindungsgemäßer Festbettreaktor ein Röhrchen zur Bestimmung von kurzkettigen Alkoholen in einem Gasgemisch ist. Beispielsweise kann der erfindungsgemäße Festbettreaktor ein Röhrchen zur Bestimmung von Methanol, Ethanol, Propanol oder Butanol sein. In jedem Fall kann das Gehäuse eines solchen Röhrchens durch eine Glasröhre gebildet werden, in welcher das Festbett entsprechend angeordnet ist. Das Festbett kann dabei zum Beispiel zwischen einer ersten und einer zweiten Haltevorrichtung in der Röhre, d.h. in dem Gehäuse angeordnet sein. Die Haltevorrichtung können zum Beispiel Drahtgewebe oder dergleichen sein. Eine Glasröhre hat in diesem Zusammenhang den Vorteil, dass sie durchsichtig ist. Eine Farbumschlagsreaktion, die in dem Festbett stattfindet, ist mithin von außen beobachtbar. Selbstverständlich ist es auch vorstellbar, dass das Gehäuse aus transparentem Kunststoff, beispielsweise aus einer Kunststoffröhre besteht.

In einer weiteren bevorzugten Ausführungsform kann ein erfindungsgemäßer Festbettreaktor eine Vorrichtung zur Herstellung von Formaldehyd und/oder Acetaldehyd sein.

Der partikelförmige Träger des Festbettes kann in jedem Fall durch Silikapartikel (Silikagel) gebildet werden. Diese Silikapartikel, d.h. der partikelförmige Träger, können bzw. kann dann mit dem reaktiven Stoff imprägniert sein. Selbst verständlich ist auch vorstellbar, dass die Silikapartikel zusätzlich mit weiteren Substanzen imprägniert sind. Dies ist jedoch nicht zwingend notwendig. Unter Silikapartikel werden in diesem Zusammenhang körnchenförmige Partikel verstanden, die aus einer Silikaverbindung bestehen. Insofern besteht der partikelförmige Träger des Festbettes aus einer Silikaverbindung. Silikapartikel weisen eine gewisse Transparenz auf, sodass ein Farbumschlag gut beobachtbar ist. Ein großer Vorteil von Silikapartikeln liegt außerdem darin, dass sie chemisch inert sind. Es eignen sich sowohl amorphes, nanoporöses als auch unporöses Siliziumdioxid. Bevorzugt ist amorphes, nanoporöses Siliziumdioxid.

Die Pyridinverbindung ist ausgewählt aus Pyridiniumchlorochromat, Pyridiniumdichromat und Bispyridinchromtrioxid, bevorzugt Pyridiniumdichromat. Insbesondere Pyridiniumdichromat zeigt bei Kontakt mit Atemalkohol einen deutlichen Farbumschlag von gelb nach grün. Weitere nachweisbare, gasförmige Verbindungen sind zum Beispiel 2-Propanol, Ethylenoxid, Dichlormethan, Vinylchlorid und/oder Chloroform. Die Verwendung von Pyridinverbindungen bietet dabei viele Vorteile. So weisen die erfindungsgemäßen Festbettreaktoren überraschenderweise eine deutlich erhöhte Langzeitlagerstabilität auf. Weiterhin kann der Nachweis bereits mit einer relativ geringen Menge reaktiven Stoffes eindeutig geführt werden. Die erfindungsgemäßen Festbettreaktoren, sind zudem überraschenderweise in einem sehr großen Temperatur- und Feuchtebereich zuverlässig einsetzbar. Insbesondere sind sie relativ unempfindlich gegenüber Wasser- bzw. Feuchteeintrag.

In diesem Zusammenhang hat es sich auch als vorteilhaft herausgestellt, wenn der Träger mit dem reaktiven Stoff imprägniert ist. Der reaktive Stoff ist mithin an der Oberfläche des Trägers angeordnet. Ein Gasstrom, der zwischen den Trägerpartikeln hindurchströmt, kann somit an dem reaktiven Stoff vorbeiströmen. Beispielsweise in dem Gasstrom enthaltener Alkohol kann dann mit der Pyridinverbindung entsprechend reagieren. Besonders günstig ist es dabei, wenn der Träger ein Träger ist, der dadurch hergestellt ist, dass Silikapartikel mit einer Imprägnierlösung versetzt und anschließend unter mechanischer Bewegung miteinander vermischt werden. Die Imprägnierlösung ist dabei eine Lösung der erfindungsgemäßen metallorganischen Pyridinverbindung in einem Lösemittel. Das Lösemittel ist Phosphorsäure. Denkbar ist dabei auch, dass das Lösemittel zusätzlich eine weitere Säure und/oder ein Säureanhydrid aufweist. Diese weitere Säure kann beispielsweise Schwefelsäure sein, das weitere Säureanhydrid kann beispielsweise Essigsäureanhydrid sein. Durch die zusätzliche Zugabe von Säureanhydrid kann die Farbgebung noch deutlich verstärkt und die Empfindlichkeit des Nachweises gesteigert werden. Das Säureanhydrid wird dabei bevorzugt in einer katalytischen Menge zugegeben. Beispielsweise ist denkbar, dass eine Menge von nicht mehr als 500 µL, bevorzugt nicht mehr als 300 µl zu einem Ansatz von 100g gegeben werden. Insbesondere für den Nachweis von alkoholischen Verbindungen in einem Gasgemisch hat es sich auch als günstig herausgestellt, wenn der Träger ein Träger ist, der dadurch hergestellt ist, dass Silikapartikel mit einer Imprägnierlösung versetzt, unter mechanischer Bewegung miteinander vermischt und anschließend getempert werden. Die Imprägnierlösung ist auch in diesem Fall ein Gemisch aus einem Lösemittel, welches Phosphorsäure und optional eine weitere Säure und/oder Säureanhydrid aufweist, mit dem reaktiven Stoff. Die weitere Säure ist z.B. Schwefelsäure, das Säureanhydrid Essigsäureanhydrid und die metallorganische Pyridinverbindung Pyridiniumdichromat.

Überraschenderweise hat sich herausgestellt, dass es gewünscht ist, wenn der Träger eine Beladung mit der metallorganischen Pyridinverbindung von höchstens 10 Gewichtsprozent, bevorzugt höchstens 5 Gewichtsprozent, besonders bevorzugt höchstens 2 Gewichtsprozent oder weniger aufweist. Man erkennt, dass ein besonderer Vorteil der erfindungsgemäßen Lösung darin liegt, dass nur eine sehr geringe Menge der metallorganischen Pyridinverbindung für einen effektiven und sicheren Nachweis benötigt wird. Dies ist insbesondere von Vorteil, wenn die Pyridinverbindung eine Chromat-Verbindung ist. Der Chromatgehalt kann dabei insgesamt deutlich ≤ 0,1% sein.

Weiterhin ist es günstig, wenn die Silikaverbindung ein Silikagel mit einer Partikelgrößenverteilung von wenigstens 0,1 mm bis höchstens 1,5 mm ist. Unter der Partikelgrößenverteilung wird dabei die Bandbreite der in dem Träger vorkommenden Größen der einzelnen Partikel verstanden. Bei einer Partikelgrößenverteilung von 0,1 mm bis 1,5 mm enthält der Träger mithin als kleinste Partikel solche mit der Größe 0,1 mm und als größte Partikel solche mit der Größe 1,5 mm. Außerdem kann der Träger Partikel mit allen dazwischen denkbaren Größen enthalten. Es versteht sich von selbst, dass verschiedene Varianten der Partikelgrößenverteilung innerhalb des erfindungsgemäßen Rahmens denkbar sind. Dabei ist es nicht notwendig, dass stets der gesamte Bereich von wenigstens 0,1 mm bis höchstens 1,5 mm abgedeckt wird. Vielmehr ist es durchaus vorstellbar, dass für verschiedene Ausführungsbeispiele jeweils nur ein Bereich innerhalb dieses genannten Bereiches realisiert wird. So ist es sowohl vorstellbar, dass der Träger ein Silikagel mit einer relativ geringen und homogenen Partikelgrößenverteilung ist, zum Beispiel zwischen 0,1 mm und 0,4 mm, bevorzugt zwischen 0,2 mm bis 0,3 mm. Denkbar ist auch ein Silikagel mit einer relativ breiten Verteilung der Partikelgrößen, beispielsweise zwischen 0,6 mm bis 1,4 mm, bevorzugt 0,8 mm bis 1,2 mm. Mithilfe der Auswahl der Partikelgrößenverteilung kann zum Beispiel Einfluss auf den Strömungswiderstand, den der Festbettreaktor einem Gasstrom entgegensetzt, genommen werden.

In einem weiteren Aspekt betrifft die Erfindung ein Verfahren zur Herstellung des Festbettes für einen Festbettreaktor gemäß einem der Ansprüche 1 bis 5, mit den Schritten:
a. Bereitstellen eines Trägers
b. Bereitstellen einer Imprägnierung
c. Behandlung des Trägers mit der Imprägnierung
wobei die Imprägnierlösung, die in Schritt b. bereitgestellt wird, eine Lösung der metallorganischen Pyridinverbindung in einem Lösemittel, welches eine anorganische Phosphor-Verbindung enthält, ist.

Insbesondere die Behandlung des Trägers mit einer Imprägnierlösung, die neben der erfindungsgemäßen metallorganischen Pyridinverbindung ein Lösemittel enthaltend Phosphorsäure aufweist, hat dabei überraschenderweise den Effekt, dass die mit dieser Lösung behandelten Trägerpartikel für eine sehr effektive Umsetzung von alkoholischen Verbindungen geeignet sind. So ist es beispielsweise auf diese Weise möglich, relativ geringe Mengen Alkohol in einer Gasprobe umzusetzen. Dabei wird überraschenderweise der enthaltene Alkohol nahezu vollständig umgesetzt, sodass ein mithilfe dieses Verfahrens hergestelltes Festbett sowohl zum quantitativen Nachweis von Alkohol als auch zur Synthese von bei der Umsetzung entstehenden Produkten, wie etwa Formaldehyd oder Acetaldehyd, eingesetzt werden kann.

Dabei bietet insbesondere die Verwendung von Phosphorsäure den großen Vorteil, dass sie bei Durchströmen des Festbettes später nicht ausgast.

In noch einer weiteren Ausführungsform ist vorgesehen, dass das Lösemittel der Imprägnierungslösung wenigstens eine weitere Säure aufweist, bevorzugt Schwefelsäure. Selbstverständlich ist es auch denkbar, dass das Lösemittel der Imprägnierlösung sowohl Schwefelsäure als auch Phosphorsäure, bevorzugt eine Mischung aus Schwefelsäure und Phosphorsäure, aufweist. Denkbar ist dabei in jedem Fall, dass das Lösemittel der Imprägnierlösung eine Mischung von Phosphorsäure zu Schwefelsäure im Verhältnis von wenigstens 3:1 und höchstens 1:3 ist. Selbst verständlich ist es auch vorstellbar, dass die Mischung Phosphorsäure und Schwefelsäure im Verhältnis von wenigstens 2:1 und höchstens 1:2 oder im Verhältnis von 1:1 vorliegt.

Es ist auch denkbar, dass das Lösemittel der Imprägnierlösung wenigstens einen Zusatz in Form eines Säureanhydrides aufweist. Das Säureanhydrid ist in einem bevorzugten Beispiel Essigsäureanhydrid, andere Säureanhydride sind selbstverständlich auch denkbar. Dabei ist es günstig, wenn der Anteil der Phosphorsäure größer ist, als der Anteil des Säureanhydrids. Es ist mithin günstig, wenn das Lösemittel der Imprägnierlösung eine Mischung von Phosphorsäure zu Essigsäureanhydrid im Verhältnis von wenigstens 50:1 und höchstens 5:1 ist. Besonders günstig ist es, wenn das Lösemittel der Imprägnierlösung eine Mischung von Phosphorsäure zu Essigsäureanhydrid im Verhältnis von wenigstens 25:1 und höchstens 15:1 ist.

In jedem Fall hat es sich als besonders günstig erwiesen, wenn das Bereitstellen der Imprägnierung das Lösen der metallorganischen Pyridiniumverbindung in einem wässrigen Lösemittel, welches wenigstens 50% Phosphorsäure enthält umfasst. Eine erfindungsgemäße Imprägnierlösung kann somit eine Imprägnierlösung sein, welche ein wässriges Lösungsmittel mit wenigstens 50 % Phosphorsäure, die metallorganische Pyridiniumverbindung und zusätzlich eine weitere Säure aufweist. Beispielsweise kann die Imprägnierlösung ein Gemisch aus einem wässrigen Lösungsmittel mit wenigstens 50 % Phosphorsäure sowie Schwefelsäure als weitere Säure und Pyridiniumchlorochromat, Pyridiniumdichromat oder Bispyridinchromtrioxid als metallorganische Pyridiniumverbindung sein. Denkbar ist dabei insbesondere, dass die Imprägnierlösung ein Gemisch ist aus einem wässrigen Lösungsmittel mit wenigstens 50 % Phosphorsäure, zusätzlich Schwefelsäure und Pyridiniumdichromat, welches in dem Gemisch aus Phosphorsäure und Schwefelsäure gelöst ist. Selbstverständlich ist aber auch vorstellbar, dass die Imprägnierlösung einen höheren Anteil an Phosphorsäure aufweist oder sogar vollständig aus Phosphorsäure besteht. Mit anderen Worten, es ist auch denkbar, dass das Bereitstellen der Imprägnierung das Lösen der metallorganischen Pyridiniumverbindung in einem wässrigen Lösungsmittel, welches mindestens zu 50% und höchstens zu 100 % Phosphorsäure enthält, umfasst.

Bevorzugt ist es in diesem Zusammenhang, wenn die Konzentration der Pyridiniumverbindung in dem Lösungsmittel wenigstens 0,001 Mol/L, bevorzugt wenigstens 0,05 Mol pro Liter, besonders bevorzugt wenigstens 0,50 Mol/L beträgt. Weiter ist es bevorzugt, dass die Konzentration der Pyridiniumverbindung in dem Lösungsmittel höchstens 0,50 Mol/L, bevorzugt höchstens 0,05 Mol/L, besonders bevorzugt höchstens 0,02 Mol/L beträgt.

Man erkennt insofern, dass die Konzentration der Pyridiniumverbindung in dem Lösungsmittel wenigstens 0,001 Mol/L und höchstens 0,50 Mol/L, bevorzugt wenigstens 0,005 Mol/L und höchstens 0,05 Mol/L, besonders bevorzugt wenigstens 0,01 Mol/L und höchstens 0,02 Mol/L beträgt. Selbst verständlich ist auch denkbar, dass die Konzentration der Pyridiniumverbindung in dem Lösungsmittel wenigstens 0,001 Mol pro Liter und höchstens 0,05 Mol pro Liter, wenigstens 0,001 Mol pro Liter und höchstens 0,1 Mol/L, wenigstens 0,005 Mol/L und höchstens 0,1 Mol/L, wenigstens 0,005 Mol/L und höchstens 0,02 Mol/L, wenigstens 0,01 Mol/L und höchstens 0,1 Mol/L oder wenigstens 0,01 Mol/L und höchstens 0,05 Mol/L beträgt.

Bei der Herstellung des erfindungsgemäßen Festbettes hat es sich weiterhin als vorteilhaft erwiesen, wenn das Behandeln des Trägers mit der Imprägnierung folgende Schritte aufweist:
- c.1: Versetzen des Trägers mit der Imprägnierung;
- c.2: Mischen des Trägers mit der Imprägnierung unter mechanischer Bewegung;
- c.3: optional: tempern des Gemisches;
- c.4: optional: erneutes Mischen des Gemisches.

Dabei ist sowohl vorstellbar, dass das Behandeln des Trägers nur die Schritte c.1 und c.2 aufweist, als auch, dass das behandeln des Trägers die Schritte c.1, c.2 und c.3 oder die Schritte c.1, c.2 und c.4 oder die Schritte c.1, c.2, c.3 und c.4 aufweist.

Das Versetzen des Trägers mit der Imprägnierlösung gemäß Schritt c.1 kann aber im einfachsten Fall erfolgen, indem die Imprägnierlösung als Flüssigkeit einfach zu dem bereitgestellten festen Träger gegeben wird. Beispielsweise kann die Imprägnierlösung in ein verschließbares Gefäß gegeben werden, indem der partikelförmige feste Träger vorgelegt ist. Dieses Gefäß kann anschließend verschlossen werden. Das Mischen des Trägers und der Imprägnierung kann dann zum Beispiel auf einem Schüttler (Schüttelmischer) erfolgen.

Das Tempern des Gemisches gemäß Schritt c.3 kann zum Beispiel bei einer Temperatur von wenigstens 50°C bis höchstens 120°C durchgeführt werden. Bevorzugt erfolgt das tempern bei einer Temperatur zwischen etwa 60°C und etwa 90°C, beispielsweise bei 80°C. Dabei ist es günstig, wenn das Tempern zum Beispiel in einem Rolltrockenschrank, d.h. unter Bewegung ausgeführt wird. Die Dauer des Schrittes c.3 kann sich dabei von wenigen Stunden bis hin zu mehreren Tagen erstrecken.

Das Mischen des Gemisches gemäß Schritt c.4 kann wiederum mithilfe des bereits zuvor genannten Schüttlers (Schüttelmischer) durchgeführt werden.

In jedem Fall ist aber vorgesehen, dass die Imprägnierlösung und der Träger in Schritt c.1 im Verhältnis von wenigstens 0,2 ml Imprägnierlösung pro 1 g Träger, bevorzugt wenigstens 0,4 ml Imprägnierlösung pro 1 g Träger, besonders bevorzugt von wenigstens 0,7 ml pro 1 g Träger, miteinander versetzt werden. Außerdem ist vorgesehen, dass die Imprägnierlösung und der Träger in Schritt c.1. im Verhältnis von höchstens 1,0 ml Imprägnierlösung pro 1 g Träger, bevorzugt höchstens 0,7 ml Imprägnierlösung pro 1 g Träger, besonders bevorzugt höchstens 0,05 ml Imprägnierlösung pro 1 g Träger, miteinander versetzt werden. Man erkennt mithin, dass es günstig ist, wenn die Imprägnierlösung und der Träger in Schritt c.1 im Verhältnis von wenigstens 0,05 ml Imprägnierlösung pro 1 g Träger und höchstens 1,0 ml Imprägnierlösung pro 1 g Träger miteinander versetzt werden. Denkbar ist auch, dass die Imprägnierlösung und der Träger in Schritt c.1 im Verhältnis von wenigstens 0,05 ml und höchstens 0,7 ml oder im Verhältnis von wenigstens 0,05 ml und höchstens 1 ml oder im Verhältnis von wenigstens 0,4 ml und höchstens 1 ml oder im Verhältnis von wenigstens 0,7 ml und höchstens 1 ml oder im Verhältnis von wenigstens 0,4 ml und höchstens 0,7 ml oder im Verhältnis von wenigstens 0,7 ml und höchstens 1,0 ml oder im Verhältnis von 0,4 ml und höchstens 1,0 ml oder im Verhältnis von wenigstens 0,7 ml und höchstens 1,0 ml Imprägnierlösung pro 1 g Träger miteinander versetzt werden.

In jeden Fall ist auf diese Weise eine Beladung mit der metallorganischen Pyridinverbindung von höchstens 10 Gewichtsprozent, bevorzugt höchstens 5 Gewichtsprozent, besonders bevorzugt höchstens 2 Gewichtsprozent oder weniger erreichbar. Ist die Imprägnierlösung dabei eine Imprägnierlösung wie oben beschrieben, d.h. weist sie eine Konzentration der Pyridiniumverbindung von wenigstens 0,001 Mol/L und höchstens 0,50 Mol/L auf, so hat sich herausgestellt, dass 1 mmol Analyt, zum Beispiel, 1 mmol Ethanol, mit einem Einsatz von weniger als ein Millimol der Pyridinverbindung umsetzbar ist. Insbesondere kann der Einsatz der Pyridiniumverbindung im molaren Verhältnis zwischen 0,25 und 0,60 mmol je 1 mmol Analyt erfolgen. Dies ist insbesondere dann der Fall, wenn die Pyridiniumverbindung Pyridiniumdichromat ist.

In einem weiteren Aspekt betrifft die Erfindung ein Gasmessröhrchen mit einem erfindungsgemäßen Festbett. Ein solches Gasmessröhrchen dient als Festbettreaktor. Es besteht typischerweise aus einem Gehäuse, in welches das Festbett eingefüllt ist. Das Gehäuse ist zum Beispiel ein Glasröhrchen mit einem ersten und einem zweiten Ende. Ein besonderer Vorteil von Glasröhrchen liegt dabei in der Transparenz des Gehäusematerials. Selbst verständlich ist auch denkbar, dass das Gehäuse aus einem anderen Material als Glas besteht, solange der in dem Festbett stattfindende Farbumschlag durch die Gehäusewand hindurch beobachtbar ist. D.h. es ist von Vorteil, wenn das Gehäuse eine transparente Wand aufweist. Durch das erste Ende kann eine zu untersuchende Gasprobe in das Glasröhrchen hineinströmen und durch das zweite Ende kann sie wieder hinausströmen. Beispielsweise kann es sich bei der zu untersuchenden Gasprobe um eine Atemgasprobe handeln. So ist vorstellbar, dass ein Proband, dessen Atem auf möglicherweise vorhandenen Alkohol hin untersucht werden soll, in ein solches Gasmessröhrchen hineinpusten soll. Selbstverständlich sind aber auch andere Anwendungen vorstellbar.

Das Festbett ist in dem Gehäuse bevorzugt zwischen zwei Halteelementen angeordnet. Denkbar ist auch das zusätzlich zu dem erfindungsgemäßen Festbett und den Halteelementen weitere Festbett-Schichten in dem Gehäuse angeordnet sind. Zum Beispiel kann eine weitere Festbett Schicht dazu dienen, Wasser aus dem zu untersuchenden Gasgemisch zu eliminieren, bevor das Gasgemisch das erfindungsgemäße Festbett durchströmt, welches mit der metallorganischen Pyridiniumverbindung imprägniert ist.

Man erkennt insofern, dass es im Sinne der Erfindung vorteilhaft ist, wenn ein Gasmessröhrchen ein Festbett aufweist, wobei das Festbett einen partikelförmigen Träger und wenigstens einen reaktiven Stoff aufweist, wobei der Träger eine Silikaverbindung ist und wobei der reaktive Stoff eine metallorganische Pyridinverbindung ist. Die Pyridinverbindung ist wie gesagt ausgewählt aus Pyridiniumchlorochromat, Pyridiniumdichromat und Bispyridinchromtrioxid, bevorzugt Pyridiniumdichromat. Insbesondere ist es günstig, wenn der Träger derart mit dem reaktiven Stoff imprägniert ist, dass der Träger eine Beladung mit der metallorganischen Pyridinverbindung von höchstens 10 Gewichtsprozent, bevorzugt höchstens 5 Gewichtsprozent, besonders bevorzugt höchstens 2 Gewichtsprozent oder weniger aufweist. Auf diese Weise kann beispielsweise eine Umsetzung von Alkohol mit einem Einsatz von ca. 0,25 mmol bis 0,6 mmol Pyridiniumdichromat pro 1 mmol nachzuweisendem Alkohol erfolgen. In einem weiteren Aspekt ist es dabei auch vorteilhaft, wenn die Silikaverbindung ein Silikagel mit einer Partikelgrößenverteilung von wenigstens 0,1 mm bis höchstens 1,5 mm ist.

Die Erfindung betrifft außerdem die Verwendung der metallorganischen Pyridiniumverbindungen, insbesondere Pyridiniumdichromat, in einem Festbettreaktor zum Nachweis von alkoholischen Verbindungen. Weiterhin betrifft die Erfindung die Verwendung der metallorganischen Pyridiniumverbindungen, insbesondere Pyridiniumdichromat, in Festbettreaktoren zur Bereitstellung von Formaldehyd und/oder Acetaldehyd. Der Einsatz der metallorganischen Pyridiniumverbindungen erfolgt in beiden Fällen entsprechend dem oben bereits ausgeführten. D.h. die metallorganische Pyridiniumverbindung wird bevorzugt auf einen Träger aus einer Silikaverbindung aufgebracht (imprägniert), sodass der Träger beispielsweise eine Beladung mit der metallorganischen Pyridinverbindung, bevorzugt mit Pyridiniumdichromat, von höchstens 10 Gewichtsprozent, bevorzugt höchstens 5 Gewichtsprozent, bevorzugt höchstens 2 Gewichtsprozent oder weniger aufweist. Der so beschichtete Träger kann dann in ein Gehäuse eines Festbettreaktors eingebracht werden, zum Beispiel durch Schüttung. Wird dann durch das Gehäuse ein Gasstrom geleitet, so kann dieser das Festbett durchströmen. In dem Gasstrom enthaltene Analyte, zum Beispiel Alkohol aber auch 2-Propanol, Ethylenoxid, Dichlormethan, Vinylchlorid oder Chloroform, können dann mit der in dem Festbett enthaltenen metallorganischen Pyridinverbindung reagieren. Dabei kommt es zum einen zur Umsetzung von Alkohol zu Formaldehyd. Zum anderen findet ein optisch nachweisbarer Farbumschlag statt, der die Anwesenheit des jeweiligen Analyten anzeigt.

Weitere Merkmale, Einzelheiten und Vorteile der Erfindung ergeben sich aus dem Wortlaut der Ansprüche, sowie aus der folgenden Beschreibung von Ausführungsbeispielen anhand der Zeichnungen. Es zeigen:
- Fig. 1: eine schematische Darstellung eines erfindungsgemäßen Festbettreaktors, nämlich eines Gasmessröhrchens mit einem erfindungsgemäßen Festbett,
- Fig. 2: eine schematische Darstellung des Ablaufs eines erfindungsgemäßen Verfahrens zur Herstellung eines erfindungsgemäßen Festbettes.

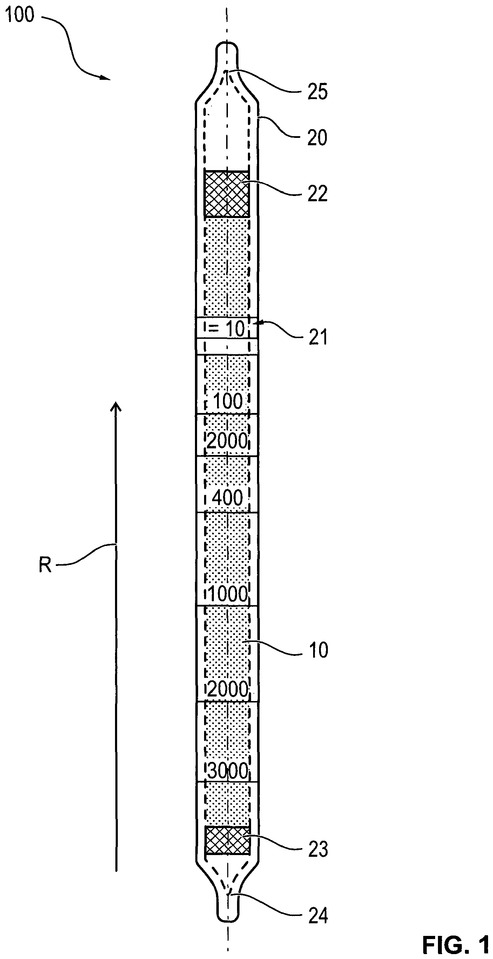

Man erkennt in Fig. 1, dass ein erfindungsgemäßer Festbettreaktor 100 Gehäuse 20 aufweist, in welchem ein erfindungsgemäßes Festbett 10 angeordnet ist. Das Gehäuse 20 ist dabei röhrenförmig mit einem ersten und einem zweiten Ende. Das erste Ende dient als Gaseinlass 25. Das zweite Ende dient als Gasauslass 24. In dem in Fig. 1 dargestellten Ausführungsbeispiel sind der Gaseinlass 25 und der Gasauslas 24 zunächst durch Glashütchen verschlossen. Diese können aufgebrochen werden, wenn der Festbettreaktor 100 verwendet werden soll. Sind der Gaseinlass 25 und der Gasauslas 24 geöffnet, so kann eine Gasprobe durch den Gaseinlass 25 in den Festbettreaktor 100 hineinströmen, das Festbett 10 durchströmen und durch den Gasauslass 24 wieder aus dem Festbettreaktor 100 herausströmen. Auf dem Gehäuse ist eine Skala 21 aufgebracht. Bei einer Reaktion von in der Gasprobe enthaltenen Analyten mit dem reaktiven Stoff des Festbettes 10 kommt es zu einem Farbumschlag. Dabei erfolgt der Farbumschlag zunächst in der Nähe des Gaseinlass 25 und setzt sich dann in Richtung des Gasauslass 24 fort. Je nach der Konzentration, in welcher der Analyt in der Gasprobe enthalten ist, ist die Länge der Strecke in Richtung R (muss in der Abbildung um 180° gedreht werden, da Durchströmungsrichtung von oben nach unten), in welcher sich der Farbumschlag ausbreitet kürzer oder länger. Mithilfe der Skala 21 kann dann die Länge dieser Strecke bestimmt und auf die enthaltene Konzentration des Analyten zurückgeschlossen werden.

Das Festbett 10 liegt als Schüttung in dem Gehäuse 20 vor. Es wird mithilfe eines ersten Halteelementes 23 und eines zweiten Halteelementes 22 in dem Gehäuse 20 festgehalten. Das Festbett 10 besteht aus einem partikelförmigen Träger, der einen reaktiven Stoff aufweist. Der Träger besteht aus Silikapartikeln, die eine Größe zwischen wenigstens 0,1 mm und höchstens 1,5 mm aufweisen. Die Silikapartikel sind mit dem reaktiven Stoff imprägniert. Der reaktive Stoff ist eine metallorganische Pyridinverbindung. In einem ersten Ausführungsbeispiel ist der reaktive Stoff Pyridiniumdichromat. Dabei weist der Träger eine Beladung von höchstens 2 Gewichtsprozent mit dem Pyridiniumdichromat auf. In jedem Fall ist das Festbett 10 dadurch hergestellt, dass der Träger mit einer Imprägnierlösung behandelt wurde, welche eine Lösung einer metallorganischen Pyridinverbindung, nämlich Pyridiniumdichromat, in einem Lösemittel ist, wobei das Lösemittel eine anorganische Phosphorverbindung, nämlich Phosphorsäure, enthält.

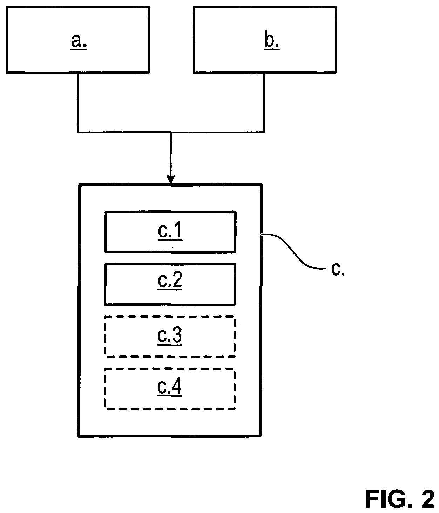

Man erkennt in Fig. 2 eine schematische Darstellung eines solchen Verfahrens zur Herstellung eines entsprechenden Festbettes 10. Das Verfahren weist die Schritte auf
a. Bereitstellen eines Trägers,
b. Bereitstellen einer Imprägnierung und
c. Behandlung des Trägers mit der Imprägnierung.

Dabei ist die Imprägnierung, die in Schritt b. bereitgestellt wird, eine Lösung der metallorganischen Pyridinverbindung in einem Lösemittel, welches eine Phosphorsäure enthält. Die metallorganische Pyridinverbindung ist in einem Ausführungsbeispiel Pyridiniumdichromat, die anorganische Phosphorverbindung ist Phosphorsäure. Weiter enthält die Imprägnierlösung (die Imprägnierung) in einem Ausführungsbeispiel eine weitere Säure, nämlich Schwefelsäure. Dabei liegen die Schwefelsäure und die Phosphorsäure im Verhältnis 1:1 vor. Im vorliegenden Ausführungsbeispiel sind neben der Schwefelsäure und der Phosphorsäure, die jeweils als wässrige Säuren vorliegen keine weiteren Bestandteile im Lösemittel der Imprägnierlösung vorhanden, d. h. die Imprägnierlösung hat ein wässriges Lösemittel, welches wenigstens 50% Phosphorsäure enthält.

In einer weiteren Variante ist die metallorganische Pyridinverbindung ebenfalls Pyridiniumdichromat, die anorganische Phosphorverbindung ist Phosphorsäure. Weiter enthält die Imprägnierlösung (die Imprägnierung) in diesem Ausführungsbeispiel ein Säureanhydrid, nämlich Essigsäureanhydrid. Dabei liegen das Essigsäureanhydrid und die Phosphorsäure im Verhältnis 1:20 vor. Auch hier hat die Imprägnierlösung ein wässriges Lösemittel, welches wenigstens 50% Phosphorsäure enthält.

Die Behandlung des Trägers mit der Imprägnierung entsprechend Schritt c. weist die Schritte c.1 und c.2 auf. In Schritt c.1 wird der Träger mit der Imprägnierung versetzt. Gemäß Schritt c.2 wird der Träger mit der Imprägnierung unter mechanischer Bewegung gemischt. Optional weist der Schritt c. Außerdem die unter Schritte c.3 und c.4 auf. In Schritt c.3 wird das Gemisch aus Träger und Imprägnierung getempert, d.h. es wird für eine gewisse Zeit einer Temperatur von wenigstens 50°C bis höchstens 120°C ausgesetzt, wobei beides gleichzeitig weiterbewegt werden kann. Beispielsweise kann das tempern in einem Rolltrockenschrank ausgeführt werden. In Schritt C4 wird das gemischte und getemperte Gemisch nochmals gemischt. Dabei kühlt das Gemisch ab.

### Beispiel 1

Entsprechend dem Schritt a. des erfindungsgemäßen Verfahrens wird ein Träger bereitgestellt, indem 100 g eines amorphen, nanoporösen Siliziumdioxides mit einer Partikelgrößenverteilung zwischen 0,8 - 1,2 mm in ein passendes Gefäß eingewogen werden.

Gemäß Schritt b. wird eine Imprägnierung bereitgestellt, indem 0,37 g Pyridiniumdichromat in einem Lösungsmittel, welches aus 44,0 ml H₂SO₄ und 44,0 ml H₃PO₄ besteht, gelöst werden.

Der Träger wird dann entsprechend Schritt c. Des erfindungsgemäßen Verfahrens mit der Imprägnierung versetzt, in dem die Imprägnierung zu dem Träger in das Gefäß gegeben wird. Die so hergestellte Mischung wird für 20 Minuten auf einem Schüttelmischer gemischt.

Im Anschluss wird das so hergestellte Festbett 10 in ein Glasröhrchen gefüllt, sodass ein Festbettreaktor 100 mit einem Festbett 10 entsteht, nämlich ein Gasmessröhrchen.

### Beispiel 2

Entsprechend dem Schritt a. des erfindungsgemäßen Verfahrens wird ein Träger bereitgestellt, indem 100 g eines amorphen, nanoporösen Siliziumdioxides mit einer Partikelgrößenverteilung zwischen 0,2 - 0,3 mm in ein passendes Gefäß eingewogen werden.

Gemäß Schritt b. wird eine Imprägnierung bereitgestellt, indem 0,25 g Pyridiniumdichromat in einem Lösungsmittel, welches aus 6,0 ml H₃PO₄ besteht, gelöst werden.

Der Träger wird dann entsprechend Schritt c. Des erfindungsgemäßen Verfahrens mit der Imprägnierung versetzt, in dem die Imprägnierung zu dem Träger in das Gefäß gegeben wird. Die so hergestellte Mischung wird für 20 Minuten auf einem Schüttelmischer gemischt. Im Anschluss wird das Gemisch für mehrere Tage bei einer Temperatur von 80 °C in einem Rolltrockenschrank getempert. Danach wird das Gemisch wiederum auf dem Schüttelmischer bei Raumtemperatur gemischt, bis es eher kalt ist.

Im Anschluss wird das so hergestellte Festbett 10 in ein Glasröhrchen gefüllt, sodass ein Festbettreaktor 100 mit einem Festbett 10 entsteht, nämlich ein Gasmessröhrchen.

### Beispiel 3

Entsprechend dem Schritt a. des erfindungsgemäßen Verfahrens wird ein Träger bereitgestellt, indem 100 g eines amorphen, nanoporösen Siliziumdioxides mit einer Partikelgrößenverteilung zwischen 0,2 - 0,3 mm in ein passendes Gefäß eingewogen werden.

Gemäß Schritt b. wird eine Imprägnierung bereitgestellt, indem 0,25 g Pyridiniumdichromat in einem Lösungsmittel, welches aus 6,0 ml H₃PO₄ und 284 µl Essigsäureanhydrid besteht, gelöst werden.

Der Träger wird dann entsprechend Schritt c. Des erfindungsgemäßen Verfahrens mit der Imprägnierung versetzt, in dem die Imprägnierung zu dem Träger in das Gefäß gegeben wird. Die so hergestellte Mischung wird für 20 Minuten auf einem Schüttelmischer gemischt. Im Anschluss wird das Gemisch für mehrere Tage bei einer Temperatur von 80 °C in einem Rolltrockenschrank getempert. Danach wird das Gemisch wiederum auf dem Schüttelmischer bei Raumtemperatur gemischt, bis es eher kalt ist.

Im Anschluss wird das so hergestellte Festbett 10 in ein Glasröhrchen gefüllt, sodass ein Festbettreaktor 100 mit einem Festbett 10 entsteht, nämlich ein Gasmessröhrchen. Die Erfindung ist nicht auf eine der vorbeschriebenen Ausführungsformen beschränkt, sondern in vielfältiger Weise abwandelbar.

### Bezugszeichenliste

- R: Richtung

- 10: Festbett
- 100: Festbettreaktor
- 20: Gehäuse
- 21: Skala
- 22: Halteelement
- 23: Halteelement
- 25: Gaseinlass
- 24: Gasauslass

## Patentansprüche

1. Festbett (10) für einen Festbettreaktor (100), wobei das Festbett (10) einen partikelförmigen Träger und wenigstens einen reaktiven Stoff aufweist und wobei
der Träger eine Silikaverbindung ist, wobei
der reaktive Stoff eine metallorganische Pyridinverbindung ist,
die metallorganische Pyridinverbindung ausgewählt ist aus Pyridiniumchlorochromat, Pyridiniumdichromat und Bispyridinchromtrioxid;
der Träger mit einer Imprägnierung imprägniert ist und die Imprägnierung erhältlich ist durch ein Verfahren umfassend den Schritt des Behandelns des Trägers mit einem Lösemittel umfassend die metallorganische Pyridiniumverbindung in dem Lösemittel gelöst und das Lösemittel Phosphorsäure,
insbesondere ein wässriges Lösemittel, welches wenigstens 50% Phosphorsäure enthält, umfasst; und
der Träger eine Beladung mit der metallorganischen Pyridinverbindung von höchstens 10 Gewichtsprozent aufweist.

2. Festbett nach Anspruch 1, **dadurch gekennzeichnet, dass** die metallorganische Pyridinverbindung Pyridiniumdichromat ist.

3. Festbett nach einem der vorhergehenden Ansprüche, **dadurch gekennzeichnet, dass** der Träger eine Beladung mit der metallorganischen Pyridinverbindung von höchstens 5 Gewichtsprozent, bevorzugt höchstens 2 Gewichtsprozent oder weniger aufweist.

4. Festbett nach einem der vorhergehenden Ansprüche, **dadurch gekennzeichnet, dass** die Silikaverbindung ein Silikagel mit einer Partikelgrößenverteilung von wenigstens 0,1 mm bis höchstens 1,5 mm ist.

5. Festbett nach Anspruch 1, **dadurch gekennzeichnet, dass** das Lösemittel wenigstens eine weitere Säure und/oder ein Säureanhydrid aufweist, bevorzugt Schwefelsäure.

6. Verfahren zur Herstellung eines Festbettes für einen Festbettreaktor gemäß einem der vorhergehenden Ansprüche, mit den Schritten:
a. Bereitstellen eines Trägers,
b. Bereitstellen einer Imprägnierung, wobei das Bereitstellen der Imprägnierung das Lösen der metallorganische Pyridiniumverbindung in einem Lösemittel umfasst und das Lösemittel Phosphorsäure, insbesondere ein wässriges Lösemittel, welches wenigstens 50% Phosphorsäure enthält, umfasst,
c. Behandlung des Trägers mit der Imprägnierung.

7. Verfahren nach Anspruch 6, **dadurch gekennzeichnet, dass** das Lösemittel der Imprägnierung wenigstens eine weitere Säure und/oder ein Säureanhydrid aufweist, bevorzugt Schwefelsäure.

8. Verfahren nach Anspruch 6 oder 7, **dadurch gekennzeichnet, dass** das Behandeln des Trägers mit der Imprägnierung folgende Schritte aufweist:
c.1 Versetzen des Trägers mit der Imprägnierung;
c.2 Mischen des Trägers mit der Imprägnierung unter mechanischer Bewegung;
c.3 optional: tempern des Gemisches;
c.4 optional: erneutes Mischen des Gemisches.

9. Verfahren nach einem der Ansprüche 6 bis 8, **dadurch gekennzeichnet, dass** die Imprägnierung in Form einer Imprägnierlösung und der Träger in Schritt c.1 im Verhältnis von wenigstens 0,05 ml Imprägnierlösung pro 1 g Träger, bevorzugt wenigstens 0,4 ml Imprägnierlösung pro 1 g Träger, besonders bevorzugt von wenigstens 0,7 ml pro 1 g Träger, miteinander versetzt werden.

10. Verfahren nach einem der Ansprüche 6 bis 8, **dadurch gekennzeichnet, dass** die Imprägnierung in Form einer Imprägnierlösung und der Träger in Schritt c.1. im Verhältnis von höchstens 1,0 ml Imprägnierlösung pro 1 g Träger, bevorzugt höchstens 0,7 ml Imprägnierlösung pro 1 g Träger, besonders bevorzugt höchstens 0,05 ml Imprägnierlösung pro 1 g Träger, miteinander versetzt werden.

11. Gasmessröhrchen (100) mit einem Festbett (10) entsprechend einem der Ansprüche 1 bis 5.

12. Verwendung von metallorganischen Pyridiniumverbindungen, insbesondere Pyridiniumdichromat, in einem Festbett (10) entsprechend einem der Ansprüche 1 bis 5 als Teil eines Festbettreaktors zum Nachweis von alkoholischen Verbindungen.

13. Verwendung eines Festbetts (10) entsprechend einem der Ansprüche 1 bis 5 in Festbettreaktoren zur Bereitstellung von Formaldehyd und/oder Acetaldehyd.

## Claims

1. Fixed bed (10) for a fixed-bed reactor (100), wherein the fixed bed (10) includes a particulate support and at least one reactive substance and wherein the support is a silica compound, wherein
the reactive substance is a metal-organic pyridine compound,
the metal-organic pyridine compound is selected from pyridinium chlorochromate, pyridinium dichromate and bis(pyridine)chromium trioxide;
the support is impregnated with an impregnation and the impregnation is obtainable by a process comprising the step of treating the support with a solvent comprising the metal-organic pyridinium compound dissolved in the solvent and the solvent comprises phosphoric acid, especially an aqueous solvent that contains at least 50% phosphoric acid; and
the support has a loading with the metal-organic pyridine compound of not more than 10 percent by weight.

2. Fixed bed according to Claim 1, **characterized in that** the metal-organic pyridine compound is pyridinium dichromate.

3. Fixed bed according to either of the preceding claims, **characterized in that** the support has a loading with the metal-organic pyridine compound of not more than 5 percent by weight, preferably not more than 2 percent by weight or less.

4. Fixed bed according to any of the preceding claims, **characterized in that** the silica compound is a silica gel having a particle size distribution of from not less than 0.1 mm to not more than 1.5 mm.

5. Fixed bed according to Claim 1, **characterized in that** the solvent includes at least one further acid and/or an acid anhydride, preferably sulfuric acid.

6. Process for producing a fixed bed for a fixed-bed reactor according to any of the preceding claims, comprising the steps of:
a. providing a support,
b. providing an impregnation, where the provision of the impregnation comprises dissolving the metal-organic pyridinium compound in a solvent and the solvent comprises phosphoric acid, especially an aqueous solvent that contains at least 50% phosphoric acid,
c. treating the support with the impregnation.

7. Process according to Claim 6, **characterized in that** the solvent of the impregnation includes at least one further acid and/or an acid anhydride, preferably sulfuric acid.

8. Process according to Claim 6 or 7, **characterized in that** the treatment of the support with the impregnation has the following steps:
c.1 admixing the support with the impregnation;
c.2 mixing the support with the impregnation under mechanical agitation;
c.3 optionally: heating the mixture;
c.4 optionally: further mixing of the mixture.

9. Process according to any of Claims 6 to 8, **characterized in that** the impregnation in the form of an impregnation solution and the support in step c.1 are admixed with one another in a ratio of not less than 0.05 ml of impregnation solution per 1 g of support, preferably not less than 0.4 ml of impregnation solution per 1 g of support, more preferably not less than 0.7 ml per 1 g of support.

10. Process according to any of Claims 6 to 8, **characterized in that** the impregnation in the form of an impregnation solution and the support in step c.1 are admixed with one another in a ratio of not more than 1.0 ml of impregnation solution per 1 g of support, preferably not more than 0.7 ml of impregnation solution per 1 g of support, more preferably not more than 0.05 ml of impregnation solution per 1 g of support.

11. Gas measuring tube (100) containing a fixed bed (10) according to any of Claims 1 to 5.

12. Use of metal-organic pyridinium compounds, especially pyridinium dichromate, in a fixed bed (10) according to any of Claims 1 to 5 as part of a fixed-bed reactor for the detection of alcoholic compounds.

13. Use of a fixed bed (10) according to any of Claims 1 to 5 in fixed-bed reactors for providing formaldehyde and/or acetaldehyde.

## Revendications

1. Lit fixe (10) conçu pour un réacteur (100) à lit fixe, ledit lit fixe (10) comprenant un substrat particulaire et au moins une substance réactive, et ledit substrat étant un composé de silice, sachant que
la substance réactive est un composé de pyridine organométallique,
lequel composé de pyridine organométallique est sélectionné parmi le chlorochromate de pyridinium, le dichromate de pyridinium et le trioyde de chrome bispyridinium ;
le substrat est imprégné par une imprégnation, et ladite imprégnation peut être obtenue par un procédé incluant l'étape de traitement dudit substrat à l'aide d'un solvant présentant le composé de pyridinium organométallique dissous dans ledit solvant, lequel solvant contient de l'acide phosphorique, en particulier un solvant aqueux renfermant au moins 50 % d'acide phosphorique ; et
ledit substrat présente une charge en composé de pyridine organométallique de 10 pour cent en poids au maximum.

2. Lit fixe selon la revendication 1, **caractérisé par le fait que** le composé de pyridine organométallique est du dichromate de pyridinium.

3. Lit fixe selon l'une des revendications précédentes, **caractérisé par le fait que** le substrat présente une charge en composé de pyridine organométallique de 5 pour cent en poids au maximum, préférentiellement de 2 pour cent en poids au maximum, voire moins.

4. Lit fixe selon l'une des revendications précédentes, **caractérisé par le fait que** le composé de silice est un gel de silice présentant une distribution granulométrique comprise entre 0,1 mm au minimum et 1,5 mm au maximum.

5. Lit fixe selon la revendication 1, **caractérisé par le fait que** le solvant comporte au moins un autre acide et/ou un anhydride d'acide, de l'acide sulfurique de préférence.

6. Procédé de fabrication d'un lit fixe conforme à l'une des revendications précédentes et conçu pour un réacteur à lit fixe, incluant les étapes consistant à :
a. tenir un substrat à disposition,
b. tenir une imprégnation à disposition, la tenue de ladite imprégnation à disposition comprenant la dissolution du composé de pyridinium organométallique dans un solvant, lequel solvant contient de l'acide phosphorique, en particulier un solvant aqueux renfermant au moins 50 % d'acide phosphorique,
c. traiter ledit substrat par ladite imprégnation.

7. Procédé selon la revendication 6, **caractérisé par le fait que** le solvant de l'imprégnation comporte au moins un autre acide et/ou un anhydride d'acide, de l'acide sulfurique de préférence.

8. Procédé selon la revendication 6 ou 7, **caractérisé par le fait que** le traitement du substrat par l'imprégnation inclut les étapes suivantes :
c.1 combinaison du substrat avec l'imprégnation ;
c.2 mélange dudit substrat à ladite imprégnation, avec mise en mouvement mécanique ;
c.3 en option : étuvage du mélange ;
c.4 en option : amalgame réitéré dudit mélange.

9. Procédé selon l'une des revendications 6 à 8, **caractérisé par le fait que** le substrat, et l'imprégnation revêtant la forme d'une solution d'imprégnation, sont combinés mutuellement, à l'étape c.1, dans le rapport d'au moins 0,05 ml de solution d'imprégnation pour 1 g de substrat, préférentiellement d'au moins 0,4 ml de solution d'imprégnation pour 1 g de substrat, d'au moins 0,7 ml pour 1 g de substrat avec préférence particulière.

10. Procédé selon l'une des revendications 6 à 8, **caractérisé par le fait que** le substrat, et l'imprégnation revêtant la forme d'une solution d'imprégnation, sont combinés mutuellement, à l'étape c.1, dans le rapport de 1,0 ml au maximum de solution d'imprégnation pour 1 g de substrat, préférentiellement de 0,7 ml au maximum de solution d'imprégnation pour 1 g de substrat, de 0,05 ml au maximum de solution d'imprégnation pour 1 g de substrat avec préférence particulière.

11. Tube (100) de mesure de gaz, muni d'un lit fixe (10) correspondant à l'une des revendications 1 à 5.

12. Utilisation de composés de pyridinium organométalliques, notamment de dichromate de pyridinium, dans un lit fixe (10) correspondant à l'une des revendications 1 à 5 et faisant partie intégrante d'un réacteur à lit fixe dévolu à la détection de composés alcooliques.

13. Utilisation d'un lit fixe (10) correspondant à l'une des revendications 1 à 5, dans des réacteurs à lits fixes dévolus à la production de formaldéhyde et/ou d'acétaldéhyde.
